# EUROPEAN PATENT SPECIFICATION

(11) **EP 4 635 409 B1**
(45) Date of publication and mention of the grant of the patent: **18.11.2026**
(21) Application number: 25200415.5
(22) Date of filing: 18.03.2020
(51) Int. Cl.: A61B 5/145, A61B 5/00

(54) **METHOD FOR STABILIZING CONTINUOUS BLOOD GLUCOSE MONITORING SYSTEM**
VERFAHREN ZUR STABILISIERUNG EINES KONTINUIERLICHEN BLUTZUCKERÜBERWACHUNGSSYSTEMS
PROCÉDÉ DE STABILISATION D'UN SYSTÈME DE SURVEILLANCE CONTINUE DE LA GLYCÉMIE

(30) Priority: 30.07.2019 KR 20190092492
(43) Date of publication of application: 22.10.2025
(62) Document number(s) of the earlier application(s) in accordance with Art. 76 EPC: 20848138.2 / 3 984 453
(73) Proprietor: i-Sens, Inc., Seoul 06646 (KR)
(72) Inventor: LEE, David, 06646 Seoul (KR); NAH, Ji Seon, 06646 Seoul (KR); SEO, Jung Hee, 06646 Seoul (KR); KANG, Young Jea, 06646 Seoul (KR)
(74) Representative: BCKIP Part mbB

(56) References cited:
- US-A1- 2009 143 659
- US-A1- 2014 000 338
- US-A1- 2014 135 605
- US-A1- 2017 303 786

## Description

### TECHNICAL FIELD

The present invention is related to a method of operating a system comprising a communication terminal and a sensor transmitter.

### BACKGROUND

Diabetes is a major cause of death and a cause of disability worldwide, and therefore, many people have health problems due to diabetes. Specially, diabetes is a serious disease that causes heart and kidney disease, blindness, nerve damage and high blood pressure. According to a long-term clinical study, the incidence of complications can be significantly reduced by appropriately managing blood glucose levels. Therefore, it is important to continuously manage diabetes, an an important factor is self-monitoring of blood glucose levels.

In response to this demand, a self-diagnosis biometer in which a user can check a blood glucose level of the user by himself or herself has been widely distributed and used. A conventional blood glucose meter measures the blood glucose level of the user by putting the user's blood on a sensor strip, which is a test strip. Accordingly, the sensor strip with the blood is inserted into the blood glucose meter, and the blood glucose level measured through the sensor strip is displayed on the blood glucose meter.

At this time, the blood glucose meter receives an electrical signal generated by an electrochemical reaction between the collected blood and the reactant in the sensor strip, and measures the blood glucose level. Such a finger prick method helps diabetic patients to manage blood glucose, but it is difficult to accurately identify the blood glucose levels which are being frequently changed because it shows only the result at the time of the measurement.

Diabetics patients generally experience hyperglycemia and hypoglycemia, an emergency may occur in the hypoglycemic conditions, and the patients may become unconscious or die if a hypoglycemic condition lasts for an extended period of time without the supply of sugar. Accordingly, although rapid discovery of the hypoglycemic condition is critically important for diabetics, blood-collecting type glucose monitoring devices intermittently measuring glucose have limited ability to accurately measure blood glucose levels.

To overcome such a drawback, continuous glucose monitoring systems (CGMSs) inserted into the human body to measure a blood glucose level every few minutes have been developed, and therefore easily perform the management of diabetics and responses to an emergency situation.

The continuous blood glucose measurement system comprises a body attachable unit for measuring blood glucose by being inserted to human body and collecting test substances such as blood of the user, and a communication terminal for communicating with the body attachable unit and displaying the blood glucose level measured by the body attachable unit.

The sensor transmitter is inserted into the human body, and in a state that a part of a sensor included in the sensor transmitter is inserted to the human body, biometric information is measured. At that time, due to the insertion of the sensor into the human body, the body can show the viral reaction by recognizing that foreign substance invades human body. If the viral reaction occurs in the human body, the measurement of the biometric information measured through the sensor may not precisely performed.

Accordingly, before performing the measurement of the biometric information, the conventional art waits for a certain time period for stabilization (e.g. two (2) hours) after inserting a sensor into the human body so that the sensor can be stably attached to the human body. During the stabilization time period, the sensor transmitter measures biometric information of the human body, and the sensor transmitter transmits the measured biometric information to the communication terminal. At that time, because the communication terminal does not output the measured data to the user for the stabilization time period of the sensor transmitter, the user is unable to check information regarding the biometric information during the stabilization time period of the sensor transmitter.

The stabilization time period of the conventional art is set as a uniformly predetermined constant time (for example, two (2) hours). Therefore, even if the sensor transmitter is stabilized earlier than the uniformly predetermined constant time, the user cannot check the measured biometric information data during the predetermined stabilization time period.

Furthermore, even if the stabilization time period lapses, the communication terminal displays the biometric information data measured by the sensor transmitter even though the sensor transmitter is not stabilized during the predetermined stabilization time period, and therefore inaccurate biometric information data can be provided to the user.

US 2009 / 0 143 659 A1 discloses a method of use for continuous analyte measurement of a host's vascular system. A continuous glucose measurement system includes a vascular access device, a sensor and sensor electronics, and the system is configured for insertion into communication with a host's circulatory system.

US 2014 / 0 135 605 A1 discloses a method for initializing an analyte sensor, such as a glucose sensor. Where a sensor has been disconnected and reconnected, a disconnection time is determined and a sensor initialization protocol is selected based upon the disconnection time. The sensor initialization protocol may include applying a first series of voltage pulses to the sensor.

### SUMMARY OF THE INVENTION

Against this background, the present invention provides a method of operating a system comprising a communication terminal and a sensor transmitter in accordance with claim 1.

According to an embodiment of the present disclosure which is not part of the claimed invention, a stabilization method may comprise: measuring biometric information by a sensor transmitter configured to be attachable to a body part of a user and measure the biometric information; transmitting biometric information data regarding the measured biometric information from the sensor transmitter to a communication terminal; performing stabilization of the biometric information data transmitted from the sensor transmitter to the communication terminal; and after the stabilization of the biometric information data is completed, displaying the biometric information data received from the sensor transmitter so that the user is able to check the biometric information data, wherein the communication terminal variably adjusts a time period for performing the stabilization of the biometric information data transmitted from the sensor transmitter.

The performing the stabilization of the biometric information data may comprise: a first stabilization step performing the stabilization of the biometric information data received from the sensor transmitter for a first time period set by the communication terminal; and a second stabilization step performing the stabilization of the biometric information data received from the sensor transmitter for a second time period before a setting time set by the communication terminal.

The second time period for performing the second stabilization step may be determined based on the biometric information data received from the sensor transmitter while performing the first stabilization step.

The performing the stabilization of the biometric information data may further comprise, after the second stabilization step, checking whether the biometric information data received from the sensor transmitter is stabilized, and the biometric information data received from the sensor transmitter may be displayed after the second stabilization step is completed so that the user is able to check the biometric information data after the second stabilization step is completed.

The checking of whether the biometric information data is stabilized may be performed based on whether the biometric information data received from the sensor transmitter is compromised to a set range.

The performing the stabilization of the biometric information data may further comprise, if the biometric information data received from the sensor transmitter is not stabilized while performing the second stabilization step, a third stabilization step performing the stabilization of the biometric information data received from the sensor transmitter by the communication terminal.

The stabilization method may further comprise checking whether the biometric information data received from the sensor transmitter by the communication terminal is stabilized while performing the third stabilization step, wherein, if the biometric information data received from the sensor transmitter is stabilized while performing the third stabilization step, the communication terminal terminates an operation of the stabilization.

The third stabilization step may be performed not to exceed a maximum time for performing the first to third stabilization steps.

The stabilization method may further comprise, if the third stabilization step does not stabilize the biometric information received from the sensor transmitter by the maximum time for performing the first to third stabilization steps, terminating, by the communication terminal, communication connection with the sensor transmitter.

The stabilization method may further comprise, when the communication terminal terminates the communication connection with the sensor transmitter, outputting, by the communication terminal, an interface for receiving, from the user, confirmation regarding termination of the communication connection with the sensor transmitter.

The stabilization method may further comprise, if the communication terminal terminates the communication connection with the sensor transmitter, displaying, on the communication terminal, a guide indicating that the sensor transmitter cannot be used.

### Advantageous Effects of Invention

An embodiment of the present disclosure has advantageous technical effect in which accurate biometric information data can be provided to the user by variably adjust stabilization time set for stabilizing an output of biometric information data measured by a sensor transmitter depending on each of sensor transmitter.

Additionally, if the sensor transmitter is quickly stabilized, more accurate biometric information can be provided to a user rapidly.

Further, a sensor transmitter which fails to be stabilized for a certain time period can be prevented to be used, and providing inaccurate biometric information data to a user can be prevented by guiding the user to replace that sensor transmitter.

### BRIEF DESCRIPTION OF THE DRAWINGS

FIG. 1 is a schematic diagram for illustrating a continuous blood glucose measurement system for use in a method according to an embodiment of the present invention.
FIG. 2 is a figure illustrating an applicator for attaching a sensor transmitter to a human body.
FIGS. 3 and 4 are figures for illustrating a process of attaching a sensor transmitter to a human body using an applicator.
FIG. 5 is a flowchart for illustrating a method for stabilizing a continuous blood glucose monitoring system.
FIG. 6 is a block diagram for illustrating a sensor transmitter of a continuous blood glucose measuring system for use in a method according to an embodiment of the present invention.
FIG. 7 is a block diagram of a communication terminal of a continuous blood glucose monitoring system for use in a method according to an embodiment of the present invention.
FIG. 8 is a figure for illustrating a case in which a sensor transmitter is quickly stabilized by a stabilization method of a continuous blood glucose monitoring.
FIG. 9 is a figure for illustrating a process for determining whether blood glucose information data is compromised in a stabilization method of a continuous blood glucose monitoring system.
FIG. 10 is a figure for illustrating a case in which a stabilization time of a sensor transmitter is added in a stabilization method of a continuous blood glucose monitoring system.

### DESCRIPTION OF EMBODIMENTS OF THE INVENTION

With reference to enclosed drawings, preferred embodiments are described in detail. It is understood that the invention is solely defined by the scope of the appended claims.

The technical terms used in the present disclosure are only for the purpose of describing exemplary embodiments, and they are not intended to limit the present invention, which is solely defined by the appended claims. Also, unless otherwise defined, all technical terms used herein should be construed as having the same meaning as commonly understood by those skilled in the art, and should not be interpreted as being excessively inclusive or excessively restrictive. In addition, when a technical term used herein is an erroneous technical term that does not accurately represent the idea of the present invention, it should be understood as replacing the term by a technical term which can be properly understood by those skilled in the art.

Further, singular expressions used in the present specification include plural expressions unless they have definitely opposite meanings. In the present application, it shall not be construed that terms, such as "including" or "comprising", various constituent elements or steps described in the specification need to be all essentially included, and it shall be construed that some constituent elements or steps among the various constituent elements or steps may be omitted, or additional constituent elements or steps may be further included.

Also, it should be noted that the accompanying drawings are merely illustrated to easily explain the invention, and therefore, they should not be construed to limit the invention by the accompanying drawings.

Hereinafter, with reference to the enclosed drawings, a method for stabilizing a continuous blood glucose monitoring system according to an embodiment of the present invention is described in detail.

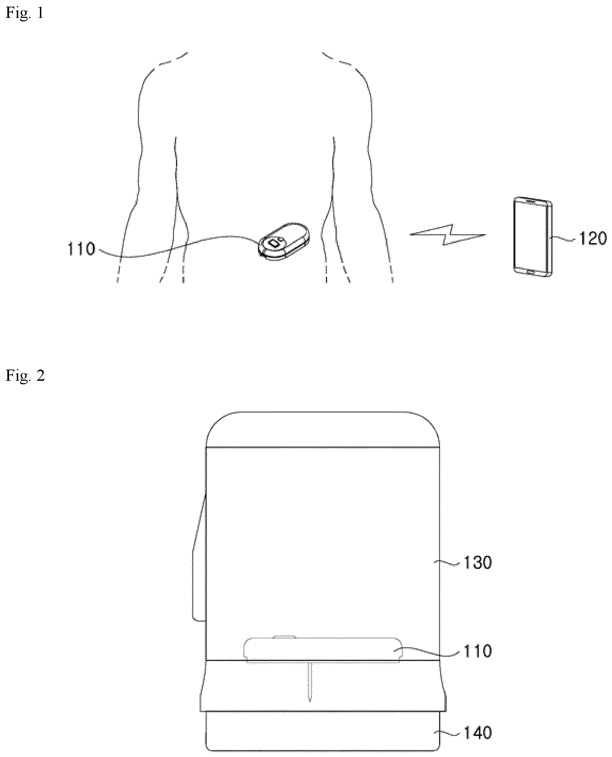

FIG. 1 is a schematic diagram for illustrating a continuous blood glucose measurement system.

Referring to FIG. 1, the continuous blood glucose measurement system comprises a sensor transmitter (110) and a communication terminal (120).

The sensor transmitter (110) is attachable to human body and, when the sensor transmitter (110) is attached to the human body, an end portion of a sensor of the sensor transmitter (110) is inserted into skin to periodically extract body fluid of the human body and measure blood glucose.

The communication terminal (120) is a terminal configured to receive blood glucose information from the sensor transmitter (110) and output or display the received blood glucose information to a user, and for example, the communication terminal (120) may be a portable terminal (such as smartphone, tablet PC, or notebook and so on) configured to communicate with the sensor transmitter (110). However, the communication terminal (120) is not limited thereto, and may be any type of a terminal which has a communication function and program or application can be installed to.

The sensor transmitter (110) transmits the blood glucose information in response to request of the communication terminal (120) or at predetermined times periodically, and for data communication between the sensor transmitter (110) and the communication terminal (120), the sensor transmitter (110) and the communication terminal (120) are communicationally connected to each other over a wire by an USB cable and so on or communicationally connected in an wireless communication means such as infrared communication, NFC communication, Bluetooth, etc.

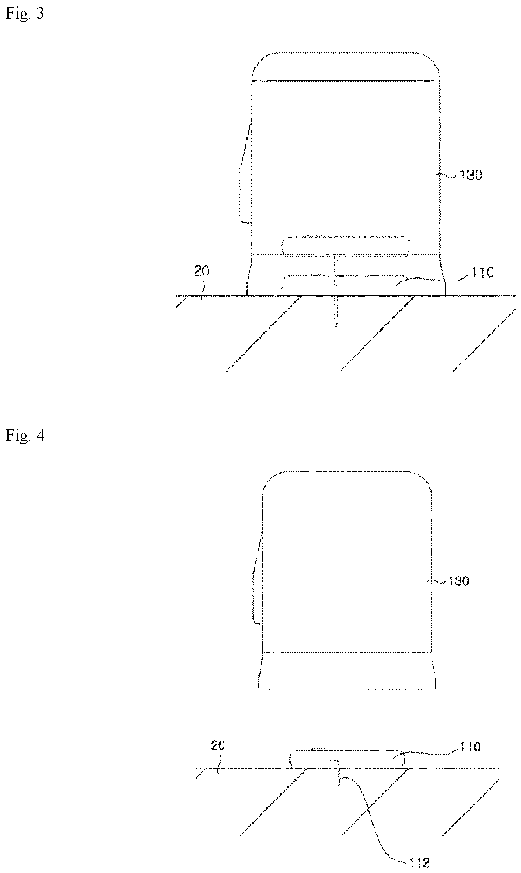

The sensor transmitter (110) is attached to a part of the human body by an applicator, FIG. 2 is a figure illustrating an applicator for attaching a sensor transmitter to a part of a human body, and FIGS. 3 and 4 are figures for illustrating a process of attaching a sensor transmitter to a human body using an applicator.

Firstly, an application (130) is now described by referring to FIG. 2, the sensor transmitter (110) is mounted in the applicator (130), and the applicator (130) can be operated so that the sensor transmitter (110) can be outwardly discharged to the outside of the applicator (130) by the manipulation of the user and then be attached to a specific portion of the human body of the user. The applicator (130) is formed to have a shape that one side of the applicator (130) is open, and the sensor transmitter (110) is installed to the applicator (130) through the open side of the applicator (130).

When the sensor transmitter (110) is attached to a part of the human body using the applicator (130), for inserting an end portion of the sensor included in the sensor transmitter (110) to skin, the applicator (130) comprises a needle (not shown) formed to cover the end portion of the sensor therein, a first elastic means (not shown) pushing the needle and the end portion of the sensor together towards the skin, and a second elastic means (not shown) configured to retract the needle only. The compressed state of the first elastic means (not shown) arranged to be compressed inside the applicator (130) by the configuration of the applicator (130) can be released, thereby inserting the needle and the end portion of the sensor simultaneously to the skin, and when the end portion of the sensor is inserted to the skin, the compressed state of the second elastic means (not shown) is released, thereby extracting the needle only. By the applicator (130), the user can safely and easily attach the sensor transmitter (110) to the skin.

A process of attaching the sensor transmitter (110) to the human body will be described in detail with reference to FIGS. 3 and 4, in a state that a protection cap (140) is separated or removed, an open side of the applicator (130) is closely placed on a specific part of skin (20) of the human body. When the applicator (130) is operated in a state that the applicator (130) is closely placed on the skin (20) of the human body, the sensor transmitter (110) is outwardly discharged from the applicator (130) and then attached to the skin (20). Here, an end portion of the sensor (112) is arranged to be exposed from the sensor transmitter (110) at a lower portion of the sensor transmitter (110), and a part of the end portion of the sensor (112) is inserted into the skin (20) by a needle installed at the applicator (130). Accordingly, the sensor transmitter (110) can be attached to the sensor (20) in a state that an end portion of the sensor (112) is inserted to the skin (20).

In the example of the present disclosure, an adhesive tape is provided at a surface of the sensor transmitter (110) contacting the human body so that the sensor transmitter (110) can be attached to the skin (20). Accordingly, if the applicator (130) is moved away from the skin (20) of the human body, the sensor transmitter (110) is fixedly attached to the skin (20) of the human body by the adhesive tape.

After that, if the power is supplied to the sensor transmitter (110), the sensor transmitter (110) is communicationally connected with the applicator (130), and the sensor transmitter (110) transmits the measured blood glucose information to the communication terminal.

The sensor transmitter (110) can measure not only the blood glucose information but also various biometric information, and hereinafter blood glucose information is illustrated as one of examples of biometric information.

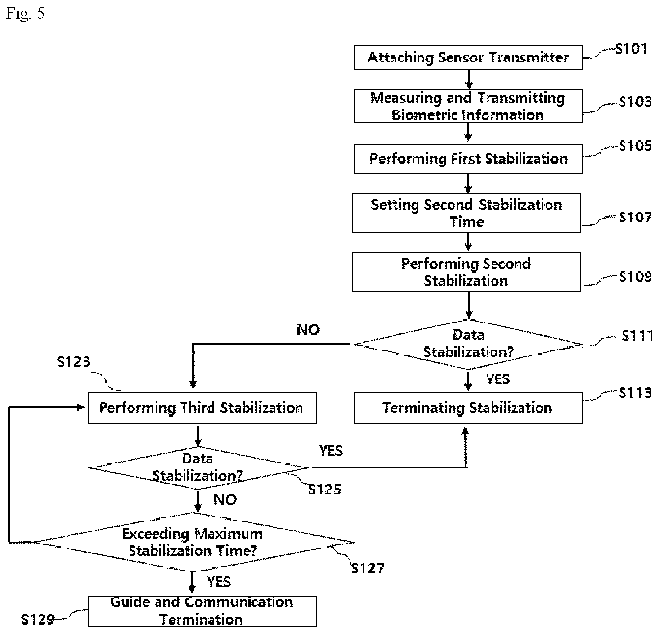

FIG. 5 is a flowchart for illustrating a method for stabilizing a continuous blood glucose monitoring system.

Referring to FIG. 5, a stabilization method of the sensor transmitter (110) of a continuous blood glucose monitoring system (100) is described. According to an example of the present disclosure, the stabilization method of the sensor transmitter (110) performs the control of stabilization time at the communication terminal (120) based on blood glucose data measured by the sensor transmitter (110). Accordingly, the stabilization method according to the present embodiment is performed at the communication terminal (120).

Firstly, the sensor transmitter (110) is attached to human body (S101).

If the sensor transmitter (110) is attached to the human body, a part of a sensor included in the sensor transmitter (110) is inserted into the human body, and if the power is supplied to sensor transmitter (110), the sensor transmitter (110) is activated and performs communication connection with the communication terminal (120).

The sensor transmitter (110) performs the monitor or measurement of the blood glucose information of the human body, and the sensor transmitter (110) transmits the measured blood glucose information to the communication terminal (120) (S103).

If the sensor transmitter (110) is attached to the human body and activated, the measurement of the blood glucose information is performed from the time of being activated. And, the blood glucose information data measured by the sensor transmitter (110) is transmitted to the communication terminal (120).

If starting to receive the blood glucose information data from the sensor transmitter (110), the communication terminal (120) performs a first stabilization step (S105).

The first stabilization step is a process in which, until the blood glucose information data is stably measured, the communication terminal (120) does not output or display the received blood glucose information data to the user, but stores the received blood glucose information data. According to the present embodiment, the first stabilization step can be performed for a predetermined time period.

The communication terminal (120) sets time for a second stabilization step using the received blood glucose information data received from the sensor transmitter (110) while the first stabilization step is performed (S107).

The communication terminal (120) can adjust the time for the second stabilization step depending on whether the blood glucose information data received from the sensor transmitter (110) at the first stabilization stage is stable, if the blood glucose information data received from the sensor transmitter (110) at the first stabilization stage is stable, the time for the second stabilization stage can be set relatively short, and if the blood glucose information data received from the sensor transmitter (110) at the first stabilization stage is unstable, the time for the second stabilization stage can be set relatively long.

And, the communication terminal (120) performs the second stabilization stage according to the set time described above (S109).

Like the first stabilization stage, the second stabilization stage is a process in which the measured blood glucose information data is received from the sensor transmitter (110) and stored, but it is not outputted or displayed to the user.

As described above, when the second stabilization stage is performed for the set time, the communication terminal (120) checks whether the blood glucose information data measured and received from the sensor transmitter (110) is stabilized (S111).

At that step, if the communication terminal (120) determines that the blood glucose information data received from the sensor transmitter (110) is stabilized, the stabilization step of the sensor transmitter (110) terminates (S113). If the stabilization step of the sensor transmitter (110) terminates, the communication terminal (120) outputs or displays the blood glucose information data received from the sensor transmitter (110) after the stabilization step of the sensor transmitter (110) terminates so that the user can check the received blood glucose information data.

However, if the communication terminal (120) determines that the blood glucose information data received from the sensor transmitter (110) is not stabilized, the communication terminal (120) perform a third stabilization stage (S123).

Like the first stabilization stage and second stabilization stage, the third stabilization stage is a process in which the measured blood glucose information data is received from the sensor transmitter (110) and stored, but it is not outputted or displayed to the user.

And, while the third stabilization stage is being performed, the communication terminal

(120) checks randomly (or at a predetermined time interval) whether the blood glucose information data received from the sensor transmitter (110) is stabilized (S125).

At this step, if the communication terminal (120) determines that the blood glucose information data is stabilized during the third stabilization stage, the communication terminal (120) moves to Step S113 to terminate the stabilization step of the sensor transmitter (110)a.

However, if the communication terminal (120) determines that the blood glucose information data received from the sensor transmitter (110) is not stabilized during the third stabilization stage, the communication terminal (120) check whether maximum stabilization time is reached (S127).

The stabilization process can be set so that the first to third stabilization steps can be performed within a predetermined time.

Accordingly, the communication terminal (120) can set maximum time for stabilization, and the maximum stabilization time means whole stabilization time for performing the first to third stabilization steps. For example, the communication terminal (120) may set the maximum time for the stabilization as three (3) hours, and if the first stabilization stage consumes one (1) hour and the second stabilization stage consumes for forty (40) minutes, then the third stabilization stage can be performed for one (1) hour and twenty (20) minutes. At the present step, the check of whether the maximum stabilization time is reached is checking whether three (3) hours which is the maximum time for the stabilization is reached.

Accordingly, if the maximum stabilization time is not reached, step S123 of the third stabilization stage continues to be performed.

And, when the maximum stabilization time is reached, the communication terminal (120) notifies to the user that the ongoing stabilization operation is not performed anymore and terminates the communication connection with that sensor transmitter (110) (S123).

At this step, the termination of the communication connection between the sensor transmitter (110) and the communication terminal (120) is a process of stopping the use of that sensor transmitter (110) because that sensor transmitter (110) cannot be used anymore.

Accordingly, because the blood glucose information data received from that sensor transmitter (110) cannot be stabilized during the stabilization time period, the communication terminal (120) terminates the communication with that sensor transmitter (110) not to use that sensor transmitter (110) which is incapable of stably measuring the blood glucose information data.

And, the communication terminal (120) displays a guide indicating that that sensor transmitter (110) cannot be used so that the user can check.

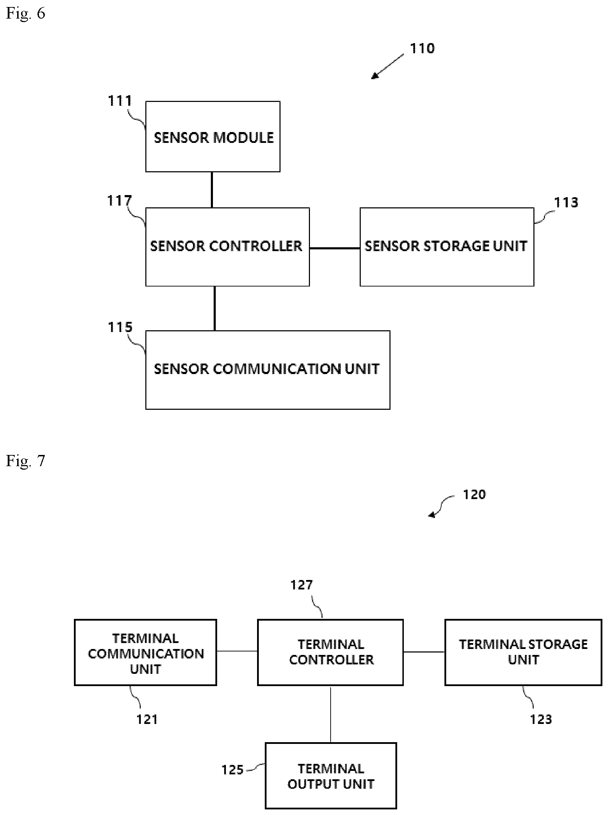

FIG. 6 is a block diagram for illustrating a sensor transmitter of a continuous blood glucose measuring system according to an example of the present disclosure.

According to an example, referring to FIG. 6, the sensor transmitter (110) comprises a sensor module (111), a sensor storage unit (113), a sensor communication unit or communicator (115) and a sensor controller (117).

The sensor module (111) comprises a sensor (112), and a part of the sensor (112) is inserted into the human body to measure blood glucose information.

The sensor storage unit (113) stores data of the blood glucose information measured by the sensor module (111). At this embodiment, the blood glucose information data stored to the sensor storage unit (113) can be stored as digital signals.

The sensor communication unit (150) transmits the blood glucose information data stored to the sensor storage unit (113) to the communication terminal (120).

The sensor controller (117) controls the sensor module (111) to measure blood glucose information at a predetermined time interval. The sensor controller (117) transforms the blood glucose information data measured as analog signals into the digital signals, and the transformed digital signals of the blood glucose information data are stored to the storage unit. Additionally, the sensor controller (117) controls the sensor communication unit (115) to transmit the stored blood glucose information data to the communication terminal (120).

FIG. 7 is a block diagram of a communication terminal of a continuous blood glucose monitoring system according to an example of the present disclosure.

According to an example, referring to FIG. 7, the communication terminal (120) includes a terminal communication unit (121), a terminal storage unit (123), a terminal output unit (125) and a terminal controller (127).

The terminal communication unit (121) communicates with the sensor communication unit (115) of the sensor transmitter (110), and receives the blood glucose information data from the sensor communication unit (115).

The terminal storage unit (123) stores the blood glucose information data received through the terminal communication unit (121).

The terminal output unit (125) outputs or displays the blood glucose information data received from the sensor transmitter (110) and stored to the terminal storage unit (123) so that the user can check the blood glucose information data. The terminal output unit (125) does not output or display the blood glucose information data being received from the sensor transmitter (110) during the stabilization operation, but outputs the blood glucose information data received from the sensor transmitter (110) after the completion of the stabilization operation.

The terminal controller (127) stores the blood glucose information data received through the terminal communication unit (121) to the terminal storage unit (123). And, the terminal controller (127) controls the terminal display unit (125) not to output or display the received blood glucose information data on the terminal display unit (125) during the stabilization operation, and performs only an operation of storing the blood glucose information data to the terminal storage unit (123). After the completion of the stabilization operation, the terminal controller (127) stores the blood glucose information data received from the sensor transmitter (110) to the terminal storage unit (123), and controls to output or display the blood glucose information data on the terminal output unit (125).

Further, the terminal controller (127) can adjust time of performing a stabilization operation of the sensor transmitter (110). The terminal controller (127) can adjust the time of performing the stabilization operation by controlling to perform stabilization for a time period set for the first stabilization stage, and setting stabilization time for the second stabilization stage and controlling to perform stabilization for the time period set for the second stabilization stage. And, the terminal controller (127) can control to perform the third stabilization stage until the maximum stabilization time is reached. As described above, the terminal controller (127) can terminate the stabilization operation in the middle of the third stabilization stage.

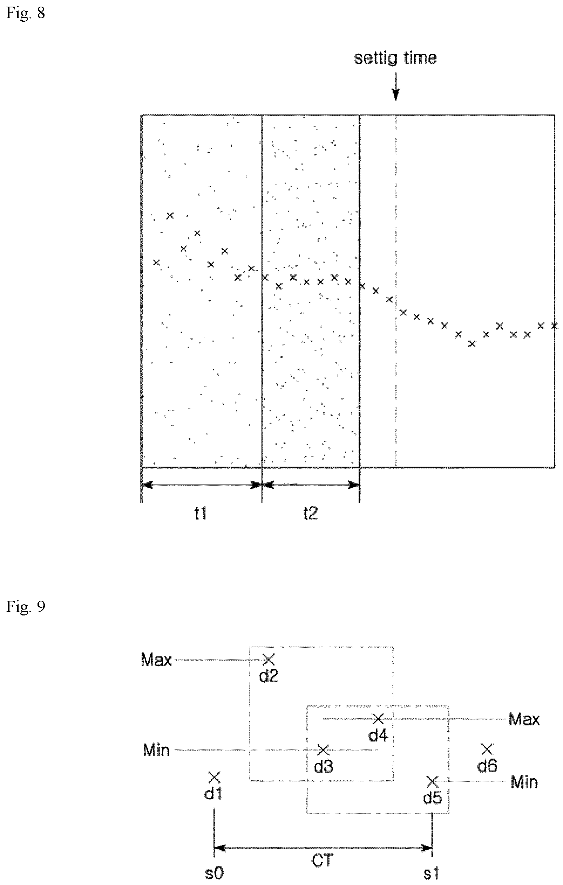

FIG. 8 is a figure for illustrating a case in which a sensor transmitter (110) is quickly stabilized by a stabilization method of a continuous blood glucose monitoring system according to an example of the present disclosure, and FIG. 9 is a figure for illustrating a process for determining whether blood glucose information data is compromised in a stabilization method of a continuous blood glucose monitoring system according to an example of the present disclosure.

Referring to FIG. 8, stabilization of blood glucose information data measured by the sensor transmitter (110) through the first stabilization stage and the second stabilization stage in the stabilization method of the present disclosure is described.

The communication terminal (120) can set that the first stabilization stage can be performed for a certain time period (t1). Accordingly, the first stabilization stage can be performed for a predetermined time period, for example, the first stabilization stage can be performed for one (1) hour.

While the first stabilization stage is being performed during the preset time period (t1), the communication terminal (120) checks whether the blood glucose information data received from the sensor transmitter (110) is within a set range. Alternatively, as described above, although the stabilization of the blood glucose information data can be checked using the blood glucose information data received by the communication terminal (120) during the set time period, but the present invention is not limited thereto, the stabilization of the blood glucose information data can be checked using at least a part of recent blood glucose information data among the received blood glucose information data.

By checking the blood glucose information data received from the sensor transmitter (110) during the first stabilization stage (t1), the communication terminal (120) can check whether the biometric signal data received by the sensor transmitter (110) with respect to time is within a certain range. As illustrated, when the blood glucose information data received by the sensor transmitter (110) is within a certain range, the communication terminal (120) can set a time period that can be consumed at the second stabilization stage as being relatively short.

Referring to FIG. 9, an exemplary embodiment of checking stabilization of blood glucose information data at the first stabilization stage is described.

The communication terminal (120) can use recent blood glucose information data among the received blood glucose information data to check whether the blood glucose information data received from the sensor transmitter (110) is within a certain range. For example, while the first stabilization stage is being performed, the communication terminal (120) can check whether a difference between a maximum value (Max) and a minimum value (Min) of three (3) latest received blood glucose information data is within a set range.

Accordingly, when d1 to d4 of the blood glucose information data are sequentially received at the first stabilization stage, whether a difference between the maximum value and the minimum value of d2 to d4 is within a set range is checked. And, if d5 of the blood glucose information data is further received, whether a difference between the maximum value and the minimum value of d3 to d5 is within the set range is checked. If the difference between the maximum value and the minimum value of d3 to d5 is within the set range, using d6 of the blood glucose information data received next, whether a difference between the maximum value and the minimum value of d4 to d6 is within the set range is still within the set range is checked.

By checking most recently received blood glucose information data, whether the blood glucose information data can be compromised can be checked by checking whether the received blood glucose information data is within a set range while performing the first stabilization stage.

Additionally, the time of the second stabilization stage can be set using time (compromise time, CT) from a time point (s0) in which blood glucose information data is received first time to a time point (s1) in which a difference between a maximum value and a minimum value of five (5) latest received blood glucose information data is compromised within a set range. Accordingly, as the compromise time (CT) is shorter, the time of the second stabilization stage is shorter, and the compromise time (CT) is longer, the time of the second stabilization stage is longer.

In the present disclosure, during the time (t2) of performing the second stabilization stage, the communication terminal (120) sets a setting time, and controls to variably perform the second stabilization stage within the setting time. For example, the communication terminal (120) can set that the first stabilization stage can be performed within one (1) hour, and if the blood glucose information data received from the sensor transmitter (110) during the first stabilization stage is compromised within a certain range, the communication terminal (120) can control a time period for the second stabilization stage so that the second stabilization stage can be performed for a time period shorter than one (1) hour which is the setting time.

In this case, the communication terminal (120) can adjust time of performing the second stabilization stage according to a degree in which the blood glucose information data received from the sensor transmitter (110) is compromised to a certain range. Accordingly, the communication terminal (120) can control the time for performing the second stabilization stage so that, if the blood glucose information data received from the sensor transmitter (110) during the first stabilization stage is compromised quickly or compromised to a narrow range in proportion to a speed of being compromised to a certain range or a size of a compromised range, the time of performing the second stabilization stage can be set shorter.

And, the communication terminal (120) can control so that the time of performing the second stabilization stage does not exceed the setting time (or a reference time). For example, the communication terminal (120) can control so that the first stabilization stage can be performed for one (1) hour, a set time period, and the second stabilization stage can be performed for maximum one (1) hour, a set time period.

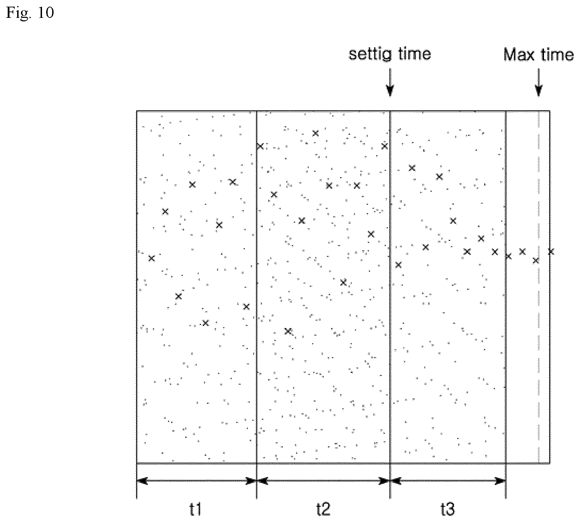

FIG. 10 is a figure for illustrating a case in which a stabilization time of a sensor transmitter is added in a stabilization method of a continuous blood glucose monitoring system.

Referring to FIG. 10, performing the stabilization of blood glucose information data measured by the sensor transmitter (110) through the first stabilization stage, the second stabilization stage and the third stabilization stage in the stabilization method of the present disclosure is described.

The communication terminal (120) can set that the first stabilization stage can be performed for a certain time period (t1). While the first stabilization stage is being performed, the communication terminal (120) checks whether the blood glucose information data received from the sensor transmitter (110) is compromised to a certain range, but as illustrated in FIG. 10, the blood glucose information data received from the sensor transmitter (110) is not comprised to the certain range. In this case, the communication terminal (120) can set time of the second stabilization stage so that the second stabilization stage can be performed until a setting time.

The time of performing the second stabilization stage is set by the communication terminal (120) as described above, and when the time consumed at the second stabilization stage is continued to the setting time, whether the blood glucose information data received from the sensor transmitter (110) is stabilized is checked. Of course, even though the second stabilization stage is not performed by the setting time, whether the blood glucose information data received from the sensor transmitter (110) is stabilized is checked.

At that time, if it is confirmed that the blood glucose information data received from the sensor transmitter (110) during the second stabilization stage is stabilized so as to be compromised to a certain range, the whole stabilization operation terminates. However, as illustrated in FIG. 10, if the stabilization so as to be compromised to a certain range does not occur, the third stabilization stage is performed.

While the third stabilization stage is being performed, the communication terminal (120) continuously checks whether the blood glucose information data received from the sensor transmitter (110) is stabilized. When the blood glucose information data received from the sensor transmitter (110) is stabilized in the middle of the third stabilization stage, the third stabilization stage terminates, and the whole stabilization operation can terminate.

When the third stabilization stage terminates, the communication terminal (120) terminates the whole stabilization operation of the sensor transmitter (110), and after the whole stabilization operation terminates, the communication terminal (120) outputs the blood glucose information data received from the sensor transmitter (110) through the terminal output (125).

However, when the stabilization of the blood glucose information data received from the sensor transmitter (110) is not completed during the third stabilization stage, the communication terminal (120) can control to perform the third stabilization stage until the maximum stabilization time Max time. By continuing to perform the third stabilization stage until the Max time, if the blood glucose information data received from the sensor transmitter (110) is stabilized, the whole stabilization operation terminates.

If the blood glucose information data received from the sensor transmitter (110) is not stabilized even though the third stabilization stage is performed until the maximum stabilization time, the communication terminal (120) determines that that sensor transmitter (110) is defective and cannot be used anymore, and immediately terminates the communication connection with that sensor transmitter (110). Alternatively, the communication terminal (120) can output or display, on the terminal output unit (125), a guide for checking whether or not the communication connection with that sensor transmitter (110) terminates to the user. When a request for terminating the communication connection between that sensor transmitter (110) and the communication terminal (120) is inputted from the user, the communication terminal (120) terminates the communication connection with that sensor transmitter (110).

If necessary, after the termination of the communication connection with the sensor transmitter (110), the blood glucose information data received through the stabilization operation from the sensor transmitter (110) can be outputted on the terminal output unit (125).

Here, if necessary, the communication terminal (120) can store information of an identifier and a serial number of the sensor transmitter (110) of which blood glucose information data is not stabilized. And, the communication terminal (120) can transmit that information to a server and so on so that the sensor transmitter (110) having that identifier and that serial number cannot be used afterward.

Additionally, as described above, if the blood glucose information data measured by the sensor transmitter (110) is not stabilized, the communication terminal (120) controls to display a guide for providing a reward for that sensor transmitter (110) to the user.

As described above, the foregoing detailed descriptions regarding the present invention have been presented by way of exemplary embodiments, but the detailed exemplary embodiments are presented as preferred examples, and therefore it should be understood that the present invention shall not be limited to those exemplary embodiments, and the scope of the present invention is solely defined by the Claims.

## Claims

1. A method of operating a system comprising a communication terminal (120) and a sensor transmitter (110), the method comprising:
receiving, by the communication terminal (120), biological information data transmitted from the sensor transmitter (110) which is configured to be attachable to a part of a user's body to measure the biological information;
determining, by the communication terminal (120), whether the biological information data transmitted from the sensor transmitter (110) has stabilized within a preset stabilization time period; and
transmitting, by the communication terminal (120), identification information of the sensor transmitter (110) to a server to prevent reuse of the sensor transmitter (110), when it is determined that the biological information data transmitted from the sensor transmitter (110) has not stabilized within the stabilization time period.

2. The method of claim 1, wherein the identification information of the sensor transmitter (110) comprises at least one selected from the group consisting of an identifier or a serial number of the sensor transmitter (110).

3. The method of claim 1 or 2, further comprising:
terminating, by the communication terminal (120), a communication connection with the sensor transmitter (110) that has not stabilized during the stabilization time period.

4. The method of any one of claims 1 to 3, further comprising:
displaying, by a terminal output unit (125) comprised in the communication terminal (120), a guide to the user to confirm whether to terminate a communication connection with the sensor transmitter (110) that has not stabilized during the stabilization time period.

5. The method of claim 4, further comprising:
terminating, by the communication terminal (120), a communication connection with the sensor transmitter (110) that has not stabilized during the stabilization time period based on user input in response to the guide displayed on the terminal output unit (125).

6. The method of claim 4 or 5, wherein the guide displayed on the terminal output unit (125) comprises information guiding the user regarding compensation for the sensor transmitter (110) that is not stabilized.

7. The method of any one of claims 1 to 6, wherein determining whether the sensor transmitter (110) is stabilized comprises:
performing, by the communication terminal (120), a first stabilization step of checking stabilization of the biological information data received from the sensor transmitter (110) during a first time period set by the communication terminal (120);
setting, by the communication terminal (120), a second time period for a second stabilization step based on the biological information data received during the first time period;
performing, by the communication terminal (120), the second stabilization step of checking stabilization of the biological information data during the second time period; and
terminating the second stabilization step when the biological information data is determined to be stabilized.

8. The method of claim 7, wherein the second time period is set based on a degree to which the biological information data received from the sensor transmitter (110) during the first time period converges within a set range.

9. The method of claim 7 or 8, further comprising:
performing, by the communication terminal (120), a third stabilization step of checking whether the biological information data received from the sensor transmitter (110) has stabilized during a third time period, if it is determined that the biological information data has not stabilized during the second stabilization step.

10. The method of claim 9, wherein, if the biological information data received from the sensor transmitter (110) is not stabilized during the third stabilization step, the communication terminal (120) determines that the biological information data from the sensor transmitter (110) has not stabilized.

11. The method of claim 9 or 10, wherein the third time period is determined such that the third stabilization step is performed not to exceed a maximum time for performing the first to third stabilization steps.

## Patentansprüche

1. Verfahren zum Betreiben eines Systems, das ein Kommunikationsendgerät (120) und einen Sensorsender (110) umfasst, wobei das Verfahren umfasst:
Empfangen, durch das Kommunikationsendgerät (120), von biologischen Informationsdaten, die von dem Sensorsender (110) übertragen werden, der so konfiguriert ist, dass er an einem Teil des Körpers eines Benutzers angebracht werden kann, um die biologischen Informationen zu messen;
Bestimmen, durch das Kommunikationsendgerät (120), ob sich die von dem Sensorsender (110) übertragenen biologischen Informationsdaten innerhalb eines voreingestellten Stabilisierungszeitraums stabilisiert haben; und
Übertragen, durch das Kommunikationsendgerät (120), von Identifikationsinformationen des Sensorsenders (110) an einen Server, um eine Wiederverwendung des Sensorsenders (110) zu verhindern, wenn bestimmt wird, dass sich die von dem Sensorsender (110) übertragenen biologischen Informationsdaten innerhalb des Stabilisierungszeitraums nicht stabilisiert haben.

2. Verfahren nach Anspruch 1, wobei die Identifikationsinformationen des Sensorsenders (110) zumindest eines, ausgewählt aus der Gruppe bestehend aus einer Kennung oder einer Seriennummer des Sensorsenders (110), umfassen.

3. Verfahren nach Anspruch 1 oder 2, ferner umfassend:
Beenden, durch das Kommunikationsendgerät (120), einer Kommunikationsverbindung mit dem Sensorsender (110), der sich während des Stabilisierungszeitraums nicht stabilisiert hat.

4. Verfahren nach einem der Ansprüche 1 bis 3, ferner umfassend:
Anzeigen, durch eine in dem Kommunikationsendgerät (120) enthaltene Endgeräteausgabeeinheit (125), einer Anleitung für den Benutzer zur Bestätigung, ob eine Kommunikationsverbindung mit dem Sensorsender (110), der sich während des Stabilisierungszeitraums nicht stabilisiert hat, beendet werden soll.

5. Verfahren nach Anspruch 4, ferner umfassend:
Beenden, durch das Kommunikationsendgerät (120), einer Kommunikationsverbindung mit dem Sensorsender (110), der sich während des Stabilisierungszeitraums nicht stabilisiert hat, auf der Grundlage einer Benutzereingabe als Reaktion auf die auf der Endgeräteausgabeeinheit (125) angezeigte Anleitung.

6. Verfahren nach Anspruch 4 oder 5, wobei die auf der Endgeräteausgabeeinheit (125) angezeigte Anleitung Informationen umfasst, die den Benutzer hinsichtlich einer Entschädigung für den Sensorsender (110), der nicht stabilisiert ist, anleiten.

7. Verfahren nach einem der Ansprüche 1 bis 6, wobei das Bestimmen, ob der Sensorsender (110) stabilisiert ist, umfasst:
Durchführen, durch das Kommunikationsendgerät (120), eines ersten Stabilisierungsschritts zum Überprüfen der Stabilisierung der von dem Sensorsender (110) empfangenen biologischen Informationsdaten während eines von dem Kommunikationsendgerät (120) festgelegten ersten Zeitraums;
Festlegen, durch das Kommunikationsendgerät (120), eines zweiten Zeitraums für einen zweiten Stabilisierungsschritt auf der Grundlage der während des ersten Zeitraums empfangenen biologischen Informationsdaten;
Durchführen, durch das Kommunikationsendgerät (120), des zweiten Stabilisierungsschritts zum Überprüfen der Stabilisierung der biologischen Informationsdaten während des zweiten Zeitraums; und
Beenden des zweiten Stabilisierungsschritts, wenn bestimmt wird, dass die biologischen Informationsdaten stabilisiert sind.

8. Verfahren nach Anspruch 7, wobei der zweite Zeitraum auf der Grundlage eines Grades festgelegt wird, in dem die während des ersten Zeitraums von dem Sensorsender (110) empfangenen biologischen Informationsdaten innerhalb eines festgelegten Bereichs konvergieren.

9. Verfahren nach Anspruch 7 oder 8, ferner umfassend:
Durchführen, durch das Kommunikationsendgerät (120), eines dritten Stabilisierungsschritts zum Überprüfen, ob sich die von dem Sensorsender (110) empfangenen biologischen Informationsdaten während eines dritten Zeitraums stabilisiert haben, wenn bestimmt wird, dass sich die biologischen Informationsdaten während des zweiten Stabilisierungsschritts nicht stabilisiert haben.

10. Verfahren nach Anspruch 9, wobei das Kommunikationsendgerät (120), wenn die von dem Sensorsender (110) empfangenen biologischen Informationsdaten während des dritten Stabilisierungsschritts nicht stabilisiert sind, bestimmt, dass sich die biologischen Informationsdaten von dem Sensorsender (110) nicht stabilisiert haben.

11. Verfahren nach Anspruch 9 oder 10, wobei der dritte Zeitraum so bestimmt wird, dass der dritte Stabilisierungsschritt durchgeführt wird, ohne eine maximale Zeit für die Durchführung des ersten bis dritten Stabilisierungsschritts zu überschreiten.

## Revendications

1. Procédé de fonctionnement d'un système comprenant un terminal de communication (120) et un émetteur de détection (110), ledit procédé comprenant :
la réception par le terminal de communication (120) de données d'informations biologiques transmises par l'émetteur de détection (110) prévu pour pouvoir être fixé sur une partie du corps d'un utilisateur afin de mesurer les informations biologiques ;
la détermination par le terminal de communication (120) si les données d'informations biologiques transmises par l'émetteur de détection (110) ont été stabilisées pendant une période de stabilisation prédéfinie ; et
la transmission par le terminal de communication (120) d'informations d'identification, de l'émetteur de détection (110) à un serveur pour empêcher la réutilisation de l'émetteur de détection (110), s'il est déterminé que les données d'informations biologiques transmises par l'émetteur de détection (110) n'ont pas été stabilisées pendant la période de stabilisation.

2. Procédé selon la revendication 1, où les informations d'identification de l'émetteur de détection (110) comprennent au moins un élément sélectionné dans le groupe constitué par un identifiant ou un numéro de série de l'émetteur de détection (110).

3. Procédé selon la revendication 1 ou la revendication 2, comprenant en outre :
la cessation par le terminal de communication (120) d'une liaison de communication avec l'émetteur de détection (110) qui n'a pas été stabilisé pendant la période de stabilisation.

4. Procédé selon l'une des revendications 1 à 3, comprenant en outre :
l'affichage, par une unité de sortie de terminal (125) comprise dans le terminal de communication (120), d'un guide pour l'utilisateur afin de confirmer s'il faut mettre fin à une liaison de communication avec l'émetteur de détection (110) qui n'a pas été stabilisé pendant la période de stabilisation.

5. Procédé selon la revendication 4, comprenant en outre :
la cessation, par le terminal de communication (120), d'une liaison de communication avec l'émetteur de détection (110) qui n'a pas été stabilisé pendant la période de stabilisation, sur la base d'une entrée d'utilisateur en réponse au guide affiché sur l'unité de sortie de terminal (125).

6. Procédé selon la revendication 4 ou la revendication 5, où le guide affiché sur l'unité de sortie de terminal (125) comprend des informations guidant l'utilisateur relativement à une compensation pour l'émetteur de détection (110) qui n'a pas été stabilisé.

7. Procédé selon l'une des revendications 1 à 6, où la détermination si l'émetteur de détection (110) est stabilisé comprend :
l'exécution, par le terminal de communication (120), d'une première étape de stabilisation où sont contrôlées les données d'informations biologiques reçues en provenance de l'émetteur de détection (110) pendant une première période fixée par le terminal de communication (120) ;
la définition, par le terminal de communication (120), d'une deuxième période pour une deuxième étape de stabilisation sur la base des données d'informations biologiques reçues pendant la première période ;
l'exécution, par le terminal de communication (120), de la deuxième étape de stabilisation où est contrôlée la stabilisation des données d'informations biologiques pendant la deuxième période ; et
la cessation de la deuxième étape de stabilisation s'il est déterminé que les données d'informations biologiques ont été stabilisées.

8. Procédé selon la revendication 7, où la deuxième période est fixée sur la base d'un degré de convergence des données d'informations biologiques reçues en provenance de l'émetteur de détection (110) pendant la première période à l'intérieur d'une plage définie.

9. Procédé selon la revendication 7 ou la revendication 8, comprenant en outre :
l'exécution, par le terminal de communication (120), d'une troisième étape de stabilisation où est contrôlée la stabilisation des données d'informations biologiques reçues en provenance de l'émetteur de détection (110) pendant une troisième période, s'il est déterminé que les données d'informations biologiques n'ont pas été stabilisées pendant la deuxième étape de stabilisation.

10. Procédé selon la revendication 9, où, si les données d'informations biologiques reçues de l'émetteur de détection (110) n'ont pas été stabilisées pendant la troisième étape de stabilisation, le terminal de communication (120) détermine que les données d'informations biologiques provenant de l'émetteur de détection (110) n'ont pas été stabilisées.

11. Procédé selon la revendication 9 ou la revendication 10, où la troisième période est déterminée de sorte que l'exécution de la troisième étape de stabilisation ne dépasse pas un temps maximum pour exécuter de la première à la troisième étape de stabilisation.
